(19) Europäisches Patentamt
European Patent Office
Office européen des brevets

(11) **EP 4 599 809 A1**

(12) **EUROPÄISCHE PATENTANMELDUNG**

(43) Veröffentlichungstag:
**13.08.2025  Patentblatt 2025/33**

(21) Anmeldenummer: **25155012.5**

(22) Anmeldetag: **30.01.2025**

(51) Internationale Patentklassifikation (IPC):
*A61F 13/537* (2006.01)        *D04H 1/4258* (2012.01)
*D04H 1/485* (2012.01)         *D04H 1/541* (2012.01)

(52) Gemeinsame Patentklassifikation (CPC):
**A61F 13/537; A61F 13/53708; D04H 1/4258;
D04H 1/485; D04H 1/541; D04H 1/5418**

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**GE KH MA MD TN**

(30) Priorität:  **07.02.2024  DE 102024103421**

(71) Anmelder: **Sandler AG
95126 Schwarzenbach/Saale (DE)**

(72) Erfinder:
• **Bernhuber, Uwe
  95032 Hof (DE)**
• **Trapp, Veronika
  95030 Hof (DE)**

(74) Vertreter: **Grünecker Patent- und Rechtsanwälte
PartG mbB
Leopoldstraße 4
80802 München (DE)**

(54) **VERFAHREN ZUR HERSTELLUNG EINES AUFNAHME- UND VERTEILVLIESSTOFFS**

(57)    Die Erfindung betrifft ein Verfahren zur Herstellung eines Aufnahme- und Verteilvliesstoff aus Stapelfasern und Saugmaterial aus regenerierter Zellulose für persönliche Hygieneprodukte, wobei der Vliesstoff aus thermoplastischen, synthetischen Stapelfaser als Stützfasern, wobei die Stützfasern homo- oder bikomponente, thermoplastische Polymerfasern sind, die schmelzbare Anteile aufweisen, Stapelfasern aus thermoplastischen und/oder duroplastischen Polymeren als Verteilfasern und Saugmaterial aus regenerierter Zellulose zusammengesetzt wird, wobei der Vliesstoff mechanisch verfestigt und anschließend thermisch mittels nachgeschalteter Heißluftverfestigung gebunden wird. Erfindungsgemäß wird der Vliesstoff aus 5 bis 35 Massenprozent an Stützfasern, 30 bis 90 Massenprozent an Verteilfasern und 5 bis 35 Massenprozent an Saugmaterial zusammengesetzt und erfolgt die mechanische Verfestigung mittels Vernadelung. Daneben betrifft die Erfindung ein nach dem erfindungsgemäßen Verfahren hergestellten Aufnahme- und Verteilvliesstoff und eine Vorrichtung zur Ausführung des erfindungsgemäßen Verfahrens.

EP 4 599 809 A1

**Beschreibung**

Die vorliegende Erfindung betrifft ein Verfahren zur Herstellung eines Aufnahme- und Verteilvliesstoffs für persönliche Hygieneprodukte gemäß dem Oberbegriff des unabhängigen Anspruchs 1.

Bei einem gattungsgemäßen Verfahren wird der Vliesstoff aus thermoplastischen, synthetischen Stapelfaser als Stützfasern, wobei die Stützfasern homo- oder bikomponente, thermoplastische Polymerfasern sind, die schmelzbare Anteile aufweisen, Stapelfasern aus thermoplastischen und/oder duroplastischen Polymeren als Verteilfasern und Saugmaterial aus regenerierter Zellulose zusammengesetzt, wobei der Vliesstoff mechanisch verfestigt und anschließend thermisch mittels nachgeschalteter thermischer Aktivierung, beispielsweise durch Heißluftverfestigung, gebunden wird.

Ein entsprechendes Verfahren ist aus der EP2692321B1 bekannt. Hierbei erfolgt die mechanische Verfestigung mit einem Wasserstrahlverfahren. Mit diesem etablierten Verfahren lassen sich Vliesstoffe mit akzeptablen Aufnahme- und Verteileigenschaften herstellen.

Derartig hergestellte Vliesstoffe werden als Teil von Hygieneprodukten verwendet, die prinzipiell folgende Bestandteile aufweisen:

- Topsheet als zum Körper hingewandte Lage

- Aufnahme- und Verteillage zur raschen Aufnahme der auftreffenden Flüssigkeit und Verteilung über die gesamte Fläche des Hygieneprodukts

- Speicherlage zur Immobilisierung der Flüssigkeitsmenge

- Backsheet als flüssigkeitsdichte Außenlage

In den letzten Jahren ging der Trend dabei immer mehr zu einer Speicherlage, welche aus superabsorbierenden Polymeren und Zellstoff besteht. Die vorgeschaltete Aufnahme- und Verteillage hat daher zum einen die Aufgabe, die Flüssigkeit zwischenzuspeichern, um einem Gelblocking der verwendeten Superabsorber vorzubeugen, muss die Flüssigkeit auch möglichst über die gesamte Fläche des Hygieneartikels verteilt werden, sodass die Speicherschicht vollständig genutzt wird.

Entsprechend besteht weiterhin der Bedarf nach einem Verfahren zur Herstellung eines Aufnahme- und Verteilvliesstoffs, das weiter verbesserte Aufnahme- und Verteileigenschaften aufweist und zudem eine verbesserte Zwischenspeicherfähigkeit und verbesserte Polstereigenschaften aufweist.

Aufgabe war es daher, ein Verfahren zur Herstellung eines Aufnahme- und Verteilvlies bereitzustellen, welches die Bereitstellung eines verbesserten Aufnahme- und Verteilvlieses mit verbesserten Aufnahme- und Verteileigenschaften, einer verbesserten Zwischenspeicherfähigkeit und verbesserten Polstereigenschaften ermöglicht.

Gelöst wird die Aufgabe anhand der Merkmale des Anspruches 1. Demnach liegt dann eine erfindungsgemäße Lösung der Aufgabe vor, wenn der Vliesstoff aus 5 bis 35 Massenprozent der Stützfaser, 30 bis 90 Massenprozent der Verteilfaser und 5 bis 35 Massenprozent des Saugmaterials zusammengesetzt wird und die mechanische Verfestigung mittels Vernadelung erfolgt.

Durch die entsprechende Zusammensetzung der Fasern und der Vernadelung als mechanische Verfestigung kann ein Aufnahme- und Verteilvlies mit verbesserten Eigenschaften mittels des erfindungsgemäßen Verfahrens hergestellt werden. So hat sich gezeigt, dass eine verbesserte Offenheit des Vliesstoffes und damit die Fähigkeit, auftreffende Flüssigkeit schnell aufzunehmen und über die Fläche des erfindungsgemäßen Vliesstoffes zu verteilen, erreicht wird, da durch die Vernadelung die Verwirbelung nur punktuell über einzelne Nadeln erreicht wird. Zusätzlich sorgen überraschenderweise die durch das Einstechen der Nadeln verursachten Stichkanäle für eine verbesserte Kapillarität, wodurch insbesondere die Verteileigenschaften des erzeugten Vlieses verbessert werden. Weiter wird eine Ausrichtung in Z-Richtung erzielt, was der Materialdicke zuträglich ist. So können überraschender Weise mit dem erfindungsgemäßen Verfahren Vliesstoffe mit höheren Materialdicken pro Flächengewicht erzeugt werden, die bei dem bekannten Verfahren auf einen sehr engen Korridor zwischen 0.12mm-0.15mm pro $10g/m^2$ begrenzt ist. Durch die höheren Materialdicken pro Flächengewicht können die Polstereigenschaften und die Zwischenspeicherfähigkeit des mittels dem erfindungsgemäßen Verfahren hergestellten Aufnahme- und Verteilvlies maßgeblich verbessert werden.

Die Begriffe Saugmaterial, Verteilfaser und Stützfaser im Sinne der Erfindung sind wie folgt definiert: Saugmaterial ist entweder ein Zellstoff oder eine regenerierte Zellulosefaser, welche aus Lösungen von Zellulosederivaten hergestellt wird, wobei die Zellulosefaser als Saugmaterial bevorzugt wird.

Der Zellstoff ist ein durch chemischen Aufschluss aus Holz gewonnenes, geflocktes Material mit einer Faserlänge von 1 bis 4 mm und kann als Saugmaterial eingesetzt werden.

Erfindungsgemäße regenerierte Zellulosefasern wie Viskose können eine Modifikation der Oberfläche haben und/oder des Querschnitts. Beispielsweise wird durch einen trilobalen Querschnitt die Saugleistung gegenüber rundem Querschnitt signifikant erhöht. Auch eine Viskose-Hohlfaser ist verwendbar.

Erfindungsgemäße Vertreter sind beispielsweise Viscostar-Viskosefasern oder normale Viskosefasern des Herstellers Lenzing, aber auch die sogenannte Galaxy-Faser der Fa. Kelheim Fibers. Übliche Faserfeinheiten liegen im Bereich von 1,0 bis 3,3 dtex, bevorzugt 1,3 bis 2,2 dtex; die eingesetzten Faserlängen sind im Bereich von 10-70mm, bevorzugt 35-50mm.

Des Weiteren können erfindungsgemäß auch regenerierten Zellulosefasern nach dem sogenannten Lyocell-Verfahren hergestellt werden, welche dadurch insbesondere im nassen Zustand eine bessere Stabilität aufweisen, so dass bei einer moderaten Flüssigkeitsaufnahme die Verteilung von Flüssigkeit begünstigt wird.

Erfindungsgemäße Vertreter sind beispielsweise Lyocell oder Tencel - Viskosefaser der Firma Lenzing. Übliche Faserfeinheiten liegen im Bereich von 1,0 bis 3,3 dtex, bevorzugt 1,3 bis 2,2 dtex; die eingesetzten Faserlängen sind im Bereich von 10-70mm, bevorzugt 35-50mm.

Auch bei dem Einsatz der erwähnten Lyocell oder Tencel- Fasern können diese nicht vollständig die Aufgabe der Flüssigkeitsverteilung übernehmen, eine Beimengung von Polyester- und/oder Polyacrylfasern innerhalb der eingesetzten Menge an Verteilfasern ist möglich.

Der Begriff "Verteilfaser" wird im Sinne der vorliegenden Erfindung für Faserstoffe aus thermoplastischen und/oder duroplastischen Polymeren verwendet, welche im erfindungsgemäßen Vliesstoff den Flüssigkeitstransport gewährleisten.

Zum Einsatz kommen dafür bevorzugt dauerhaft hydrophile Fasern aus thermoplastischen Polymeren wie Polyester oder Polypropylen. Geeignet sind aber auch duroplastische Polymere wie beispielsweise Polyacrylfasern.

Der Begriff hydrophil steht dabei für Fasern, welche im unverarbeiteten Zustand eine Sinkzeit von kleiner 5 Sekunden, bevorzugt von kleiner 3 Sekunden, aufweisen. Die Ermittlung der Sinkzeit geschieht gemäß NWSP 010.1 R0 (20).

Neben Vollfasern, d.h. Fasern, die über ihren Querschnitt vollständig aus Polymermasse bestehen, können auch Hohlfasern eingesetzt werden. Hohlfasern weisen über den Querschnitt einer Faser ein oder mehrere Hohlräume auf, sodass derartige Fasern über ihre Länge gesehen einem Schlauch ähneln. Die Faserfeinheiten liegen im Bereich von 1,7 bis 10 dtex, bevorzugt 2,2 bis 4,4 dtex; die eingesetzten Faserlängen liegen im Bereich von 10 - 80 mm, bevorzugt 35 - 60 mm. Weiterhin für die Flüssigkeitsverteilung eingesetzt werden Fasern, die keinen runden Faserquerschnitt aufzeigen, z.B. trilobal, pentalobal o.ä.

Stützfasern im Sinne der vorliegenden Erfindung sind Fasern, die in einem erfindungsgemäß aufgebauten Produkt den Faserverbund stabilisieren, sodass ein weitestgehend dimensionsstabiler Vliesstoff entsteht. Stützfasern sind homo- oder bikomponente, thermoplastische Polymerfasern, die schmelzbare Anteile aufweisen. Bei Erwärmung bis zum Erweichungspunkt schmelzen diese Anteile und stabilisieren nach dem Erkalten den Faserverbund. Erfindungsgemäß geeignete Fasern sind homopolymere Stapelfasern aus Co-Polyester, Co-Polyamid oder Polypropylen, erfindungsgemäß bevorzugt werden bikomponente Schmelzfasern in Kern-Mantel- oder Side-by-side-Anordnung aus Kombinationen von niedrigschmelzendem Co-Polyester mit Polyester, Polyethylen mit Polypropylen oder Polyethylen mit Polyester. Die Faserfeinheiten liegen im Bereich von 1,7 bis 4,8 dtex, bevorzugt 2,2 bis 4,4 dtex; die eingesetzten Faserlängen liegen im Bereich von 10 - 80 mm, bevorzugt 35 - 60 mm.

Vorteilhafte Ausführungsformen der vorliegenden Erfindung sind Gegenstand der Unteransprüche.

Gemäß einer weiteren bevorzugten Ausführungsform werden die Fasern des Vliesstoffs vor der mechanischen Verfestigung homogen miteinander vermischt und mittels eines Kardierverfahrens zu einem Faserflor geformt. Vorzugsweise erfolgt die homogene Vermischung mittels Ballenöffnungs- und Mischeinrichtungen.

In einer weiteren besonders bevorzugten Ausführungsform der vorliegenden Erfindung wird der Faserflor in Längsrichtung abgelegt. Dadurch kann ein Aufnahme- und Verteilvliesstoff hergestellt werden, dass die nötige Längsstabilität zur Verwendung für Hygieneprodukte aufweist.

In einer anderen bevorzugten Ausführungsform der vorliegenden Erfindung wird der Faserflor als ein Lagenaufbau abgelegt, der eine erste Schicht und eine zweite Schicht aufweist, wobei die erste Schicht in Längsrichtung gelegt wird und die zweite Schicht in Querrichtung oder mit einer isotropen Ausrichtung gelegt wird und auf oder unter die erste Schicht gelegt wird. Dadurch kann ein Aufnahme- und Verteilvliesstoff hergestellt werden, dass die nötige Längsstabilität zur Verwendung für Hygieneprodukte aufweist.

Vorzugsweise weist der Lagenaufbau zwei erste Schichten auf, wobei die zweite Schicht zwischen die zwei ersten Schichten geführt wird. Auch dadurch kann ein Aufnahme- und Verteilvliesstoff hergestellt werden, dass die nötige Längsstabilität zur Verwendung für Hygieneprodukte aufweist. Zusätzlich weisen die hergestellten Aufnahme- und Verteilvliesstoffe besonders vorteilhafte Polstereigenschaften auf.

Weiter vorzugsweise weisen die ersten Schichten jeweils einen Flächenmassenanteil von 25-40 % und die zweite Schicht einen Flächenmassenanteil von 20 bis 50% auf. Dies verbesset die vorteilhaften Polstereigenschaften des hergestellten Aufnahme- und Verteilvliesstoffes.

Gemäß einer besonders bevorzugten Ausführungsform der vorliegenden Erfindung erfolgt die Vernadelung mit mindestens 200 wirksamen Kerben/cm$^2$ Vliesstoff, vorzugsweise mit 200 bis 1000 wirksamen Kerben/cm$^2$ Vliesstoff, weiter vorzugsweise mit 300 bis 600 wirksamen Kerben/cm$^2$ Vliesstoff, besonders bevorzugt mit 400 bis 500 wirksamen Kerben/cm$^2$ Vliesstoff. Die wirksamen Kerben/cm$^2$ Vliesstoff definieren die Vernadelungsintensität. Sie ergeben sich aus der Anzahl der Kerben pro Nadel, der Einstichtiefe und damit der aktiven Kerben sowie der Einstichdichte. Wenn die verwendeten Nadeln zum Beispiel 6 Kerben aufweisen und 100 Einstiche/cm$^2$ Vliesstoff bei der Vernadelung mit einer Versenkung aller Kerben erfolgen, ergeben sich 600 wirksame Kerben/cm$^2$ Vliesstoff. Überraschender Weise hat sich gezeigt, dass sich bei der beanspruchten Anzahl an wirksamen Kerben/cm$^2$ Vliesstoff verbesserte Flüssigkeitsaufnahme- und Verteileigenschaften des Vliesstoffes ergeben und das Vlies gleichzeitig einen guten Polstereffekt innehat.

$$E_d = \frac{N_a \cdot n}{v}$$

$E_d$    - Einstichdichte [1/cm$^2$]
$N_a$    - spezifische Nadelanzahl [1/cm]
n    - Hubfrequenz [1/min]
v    - Durchlaufgeschwindigkeit [cm/min]

*Abbildung 1: Formel zur Errechnung der Einstichdichte, Quelle: Fuchs H., Albrecht W,: Vliesstoffe, Wiley-VCH Verlag, 2012*

Weiter vorzugsweise weisen einzelne zur Vernadelung verwendete Nadeln zwischen 1 und 9, vorzugsweise zwischen 3 und 6, Kerben auf. Durch diese Anzahl an Kerben ergibt sich eine vorteilhafte Verwirbelung im Vliesstoff.

Ebenfalls vorzugsweise weisen zur Vernadelung verwendete Nadeln einen Durchmesser zwischen 30 und 46 Gauge, vorzugsweise zwischen 38 und 40 Gauge im Arbeitsteil der Nadel und eine Gesamtlänge von 2 bis 3 Zoll auf. Die Angabe des Durchmessers erfolgt mit Maßzahlen nach dem Gauge-System (Albrecht, Fuchs, Kittelmann: Vliesstoffe. Wiley-VCH Verlag, 2000, S.291). Der Querschnitt des Arbeitsteils sowie Anordnung und Abstand der Kerben sind dabei unerheblich. Bei der Verwendung derartiger Nadeln hat sich überraschender Weise gezeigt, dass die verursachten Stichkanäle für eine verbesserte Kapillarität sorgen und sich dabei aber keine Verschlechterung der Flüssigkeitsverteilung ergibt.

In einer weiteren besonders bevorzugten Ausführungsform der vorliegenden Erfindung erfolgt die Vernadelung mit mindestens zwei, vorzugsweise vier, Nadelbrettern, die beidseitig alternierend in das einlaufende Vlies einstechen, wobei die Nadelbretter vorzugsweise eine Nadeldichte von 12000 bis 80000 Nadeln/m Arbeitsbreite, besonders bevorzugt 20000 bis 50000 Nadeln/m Arbeitsbreite aufweisen. Durch die beidseitige Vernadelung kann eine gleichmäßige Kapillarität des hergestellten Vliesstoffs erreicht werden, wodurch eine verbesserte Flüssigkeitsverteilung erreicht wird.

In einer weiteren bevorzugten Ausführungsform erfolgt die thermische Aktivierung mittels Heißluftverfestigung, wobei der Vliesstoff vorzugsweise während der Heißluftverfestigung für mindestens 20 Sekunden auf eine Temperatur oberhalb des Schmelzpunktes der schmelzbaren Anteile der Stützfasern erhitzt wird. Dadurch gibt sich eine vorteilhafte Verfestigung der hergestellten Aufnahme- und Verteilvliesstoffe.

In einer anderen bevorzugten Ausführungsform erfolgt die thermische Aktivierung mittels Infrarotwellen.

Gemäß einer bevorzugten Ausführungsform weist der Vliesstoff nach der mechanischen und thermischen Verfestigung eine Materialdicke pro Flächengewicht von mindestens 0,20 mm/10g/m$^2$, besonders bevorzugst von mindestens 0,25 mm/10g/m$^2$, auf. Dadurch können Aufnahme- und Verteilvliesstoffe mit besonders vorteilhaften Zwischenspeicherfähigkeiten hergestellt werden.

In einer weiteren bevorzugten Ausführungsform wird der Vliesstoff aus 5 bis 10 Massenprozent der Stützfaser, 80 bis 90 Massenprozent der Verteilfaser und 5 bis 10 Massenprozent des Saugmaterials zusammengesetzt. Ein derartig hergestellter Vliesstoff hat besonders gute Verteileigenschaften.

Daneben ist die Erfindung auf ein Aufnahme- und Verteilvliesstoff hergestellt nach einem der oberhalb beschriebenen Ausführungsbeispiele des erfindungsgemäßen Verfahrens gerichtet. Der erfindungsgemäße Vliesstoff weist eine dem Stand der Technik überlegene Materialdicke, Weichheit, Steifheit, Flüssigkeitsverteilung sowie Flüssigkeitsrückhaltung unter Belastung auf.

Gemäß einer bevorzugten Ausführungsform weist der Vliesstoff eine Materialdicke pro Flächengewicht von mindestens 0,20 mm/10g/m$^2$, besonders bevorzugst von mindestens 0,25 mm/10g/m$^2$, auf. Dadurch weist der Aufnahme- und Verteilvliesstoff eine besonders vorteilhafte Zwischenspeicherfähigkeiten auf.

Gemäß einer bevorzugten Ausführungsform des erfindungsgemäßen Aufnahme- und Verteilvliesstoff weist der Vliesstoff ein Flächengewicht von 40 bis 150 g/m$^2$, vorzugsweise zwischen 50-100 g/m$^2$, aufweist. Derartige Aufnahme- und Verteilvliesstoff können besonders gut für Hygieneprodukte verwendet werden.

Gemäß einer weiteren bevorzugten Ausführungsform des erfindungsgemäßen Aufnahme- und Verteilvliesstoff weist der Vliesstoff mindestens einen Run In, gemessen nach der unten beschriebenen Methode, von 10%, vorzugsweise von 15 % auf. Ein derartiger Vliesstoff hat eine bevorzugte Zwischenspeicherfähigkeit.

Gemäß einer weiteren bevorzugten Ausführungsform des erfindungsgemäßen Aufnahme- und Verteilvliesstoff weist der Vliesstoff mindestens eine Quantitative Absorption vertikal nach 20s, gemessen nach der unten beschriebenen Methode, von 1500%, vorzugsweise von 2000 % auf. Ein derartiger Vliesstoff hat eine bevorzugte Aufnahmefähigkeit.

Gemäß einer weiteren bevorzugten Ausführungsform des erfindungsgemäßen Aufnahme- und Verteilvliesstoff weist der Vliesstoff eine Stauchhärte 40% - gemäß DIN ISO 3386-1, ermittelt im ersten Belastungszyklus - von maximal 15N, vorzugsweise von maximal 9N, auf. Ein derartiger Vliesstoff hat einen verbesserten Polstereffekt.

Zudem ist die Erfindung auf eine Vorrichtung zur Ausführung eines Verfahrens gemäß einem der oberhalb beschriebenen Ausführungsbeispiele des erfindungsgemäßen Verfahrens gerichtet.

Nachfolgend werden zwei erfindungsgemäße Herstellungsverfahren und die dadurch hergestellten Aufnahme- und Verteilvliesstoffe beschrieben und mit einem vorbekannten Herstellungsverfahren und dadurch hergestellten Aufnahme- und Verteilvliesstoff verglichen.

Die zugrundeliegenden Verfahrensschritte, wie die mechanische Vernadelung, eine Wasserstrahlverfestigung oder eine thermische Heißluftverfestigung können dem im Jahr 2000 bei Wiley VCH-Verlag, Weinheim erschienenen Buch "Vliesstoffe" entnommen werden.

Herstellungsverfahren gemäß Stand der Technik

Ein beispielhaftes Herstellungsverfahren gemäß dem Stand der Technik umfasst, das Bereitstellen einer Mischung von

- 60 % Verteilfasern aus hydrophilen Polyethylentherephthalatfasern mit einem Titer von 3,3 dtex und einer Stapellänge von 38 mm.

- 25 % Saugmaterial, gebildet aus einer handelsüblichen, hydrophilen Viskosefaser mit einem Titer von 1,7 dtex und einer Stapellänge von 42 mm

- 15 % Stützfasern, gebildet aus einer hydrophilen Polyethylentherephthalat / Co- Polyethylentherephthalatfaser mit einem Titer von Titer von 4,4 dtex und einer Stapellänge von 51 mm

Die Fasern werden homogen miteinander vermischt und mittels eines Kardierverfahrens zu einem Faserflor geformt, der in Längsrichtung abgelegt wird.

Der Faserflor wird dann mittels einem Wasserstrahlverfahren mit einem zweireihigen Düsenstreifen mit 40 hpi und einem Lochdurchmesser von 0,12 mm bei kumulierten Drücken der Düsenbalken zwischen 400 und 700 bar beidseitig mechanisch und anschließend thermisch mittels nachgeschalteter Heißluftverfestigung verfestigt.

Ein entsprechendes Vlies ist unter der Produktbezeichnung Sawasoft® 1236 - 50g/m² bei der Sandler AG erhältlich.

Erfindungsgemäßes Herstellungsverfahren eines Vlies 1:

Für das erste erfindungsgemäße Herstellungsverfahren wird eine Fasermischung von

- 60 % Verteilfasern aus hydrophilen Polyethylentherephthalatfasern mit einem Titer von 3,3 dtex und einer Stapellänge von 38 mm.

- 25 % Saugmaterial, gebildet aus einer handelsüblichen, hydrophilen Viskosefaser mit einem Titer von 1,7 dtex und einer Stapellänge von 42 mm

- 15 % Stützfasern, gebildet aus einer hydrophilen Polyethylentherephthalat / Co- Polyethylentherephthalatfaser mit einem Titer von 4,4 dtex und einer Stapellänge von 51 mm

bereitgestellt.

Die Fasern werden homogen miteinander vermischt und mittels eines Kardierverfahrens zu einem Faserflor geformt, der in Längsrichtung abgelegt wird.

Dieser Faserflor durchläuft dann zunächst einen Nadelstuhl der Bauart Cyclopunch OUG-2 des Herstellers Dilo. Diese sogenannte Intensiv-Vernadelung-Passage ist mit vier Nadelbrettern zu jeweils 20183 Nadeln pro Brett und Meter Arbeitsbreite ausgelegt, welche beidseitig alternierend in das einlaufende Vlies einstechen. In Summe wirken alle Nadelbrettern mit einer Nadeldichte von insgesamt 80732 Nadeln pro Meter Arbeitsbreite auf das einlaufende Vlies ein. Die Nadelbretter sind mit einem Nadeltype 15x18x40x2,5 R222 V2206 des Hersteller Groz-Beckert bestückt.

**15x18x40x2,5 R222 V 2206**

Maßzahl (Gauge) für Schaftdurchmesser
Maßzahl (Gauge) reduzierter Schaft
Maßzahl (Gauge) Arbeitsteil
Nennlänge in Zoll

Kerbenabstand
Anzahl der Kerben auf Kante

1
2
3

Besonderheiten
Oberfläche
Kerbenform
Anwendungsbereich

*Abbildung 2: Nomenklatur von Filznadeln in Anlehnung an "Vliesstofftechnik", Arbeitgeberkreises Gesamttextil, 1987, S.4.16*

Die Bezeichnung R222 definiert die Anzahl der Kerben (hier insgesamt 6) - einen wesentlichen Kennwert bei Nadeln. Im ersten erfindungsgemäßen Herstellungsverfahren erfolgt die Vernadelung mit 147 Hüben/min und einem Vorschub von 53 mm in Maschinenrichtung pro Hub. Die Einstichtiefe beträgt 6,0 mm und die Einstichdichte 150 E/cm$^2$, wodurch sich eine Vernadelungsintensität von 300 wirksame Kerben/cm$^2$ ergibt.

Das so vorverfestigte Vlies 1 wird einem nachfolgenden Wärmeprozess in der Form einer Heißluftverfestigung zur Aktivierung der schmelzbaren Anteile innerhalb der erfindungsgemäß vorhandenen Stützfasern zugeführt. Abhängig vom Typ der eingesetzten Stützfasern wird das Vlies auf Temperaturen oberhalb des Schmelzpunktes der schmelzbaren Anteile der Stützfasern erhitzt. Diese Temperatur muss für ca. 30 Sekunden im Vlies erreicht werden, um einen ausreichenden Schmelzfluss der schmelzbaren Anteile zu erreichen. Im Falle des ersten erfindungsgemäßen Herstellungsverfahrens lag die Temperatur bei 150 °C.

Abschließend wird das so erhaltene Produkt abgekühlt und kann entsprechend den Anforderungen an die Weiterverarbeitung konfektioniert werden. Das so erzeugte, erfindungsgemäße Vlies kann ein Flächengewicht im Bereich von 40 bis 150 g/m$^2$ haben, vorzugsweise 50-90g/m$^2$.

Erfindungsgemäßes Herstellungsverfahren eines Vlies 2

Für das zweite erfindungsgemäße Herstellungsverfahren wird ebenfalls eine Fasermischung von

- 60 % Verteilfasern, darunter 35 % aus hydrophilen Polyethylentherephthalatfasern mit einem Titer von 3,3 dtex und einer Stapellänge von 38 mm.

- 25 % Saugmaterial, gebildet aus einer handelsüblichen, hydrophilen Viskosefaser mit einem Titer von 1,7 dtex und einer Stapellänge von 42 mm

- 15 % Stützfasern, gebildet aus einer hydrophilen Polyethylentherephthalat / Co- Polyethylentherephthalatfaser mit einem Titer von Titer von 4,4 dtex und einer Stapellänge von 51 mm

bereitgestellt.

Die Fasern werden ebenfalls wie beim ersten erfindungsgemäßen Herstellungsverfahren beschrieben, homogen miteinander vermischt und mittels eines Kardierverfahrens zu einem Faserflor geformt. Dabei wird mithilfe von mindestens drei Krempeln ein Lagenaufbau hergestellt. Die obere und untere Lage (erste Schichten) wird in Längsrichtung kardiert und abgelegt. Die mittlere Lage (zweite Schicht) wird durch Kreuzlegen eines Krempelflores mithilfe eines handelsüblichen Kreuzlegers der Bauart Hyperlayer (HLSC) des Herstellers Dilo zwischen die beiden längsgelegten Lagen geführt, sodass ein Hybridprodukt entsteht. Die mittlere Lage hat eine isotrope Faserorientierung, während die Ober- und Unterlage längs orientiert sind.

Zu Herstellung dieser Mittellage läuft ein ca. 30g/m$^2$ Krempelflor um 90° gedreht zur Faserlaufrichtung der bereits beschriebene Längsvliese zunächst in den oben beschriebenen Kreuzleger ein. Dieser legt insgesamt 4 Einzellagen ab, welche vor dem Zusammenlaufen mit den beiden Längsvliesen (Unterlage und Oberlage mit einem Flächengewicht von jeweils 30g/m$^2$) erst über ein Vliesstreckwerk mit 200% Verzug auf 40g/m$^2$ versteckt werden. Als Vliesstrecke wird eine Vliesstrecke der Firma Dilo (Typ VST19) verwendet.

Das sogenannte Hybridprodukt besteht somit aus einer Unterlage 30g/m$^2$, einer Mittellage 40g/m$^2$ und einer Oberlage 30g/m$^2$, welche kurz vor der Vernadelung zusammengeführt werden. Dabei können die einzelnen Lagen in den Massenanteilen durchaus wie folgt variieren:

Ober und Unterlage: Flächenmassenanteil 25-40%

Mittellage: Flächenmassenanteil 20-50%

Dieser so gebildete Faserflor durchläuft dann analog dem ersten erfindungsgemäßen Herstellungsverfahren ebenfalls einen Nadelstuhl der Bauart Cyclopunch OUG-2 des Herstellers Dilo. Diese sogenannte Intensiv-Vernadelung-Passage ist ebenfalls mit vier Nadelbrettern zu jeweils 20183 Nadeln pro Brett und Meter Arbeitsbreite ausgelegt, welche beidseitig alternierend in das einlaufende Vlies einstechen. In Summe wirken alle Nadelbrettern mit einer Nadeldichte von insgesamt 80732 Nadeln pro Meter Arbeitsbreite auf das einlaufende Vlies ein. Die verwendeten Nadeln können je nach gewünschter Intensität - was Auswirkungen auf die Materialdicke hat - unterschiedlich ausgeprägt sind. In diesem Fall sind die Nadelbretter mit einer Nadeltype 15x18x40x2,5 R222 V2206 des Hersteller Groz-Beckert bestückt. Die Bezeichnung R222 definiert die Anzahl der Kerben (hier insgesamt 6) einen wesentlichen Kennwert bei Nadeln. Im zweiten erfindungsgemäßen Herstellungsverfahren erfolgt die Vernadelung mit 147 Hüben/min und einem Vorschub von 53 mm in Maschinenrichtung pro Hub. Die Einstichtiefe beträgt 6,0 mm und die Einstichdichte 150 E/cm$^2$, wodurch sich eine Vernadelungsintensität von 300 wirksame Kerben/cm$^2$ ergibt.

Das so vorverfestigte Vlies 2 wird einem nachfolgenden Wärmeprozess in der Form einer Heißluftverfestigung zur Aktivierung der schmelzbaren Anteile innerhalb der erfindungsgemäß vorhandenen Stützfasern zugeführt. Abhängig vom Typ der eingesetzten Stützfasern wird das Vlies auf Temperaturen oberhalb des Schmelzpunktes der schmelzbaren Anteile der Stützfasern erhitzt. Diese Temperatur muss für ca. 30 Sekunden im Vlies erreicht werden, um einen ausreichenden Schmelzfluss der schmelzbaren Anteile zu erreichen. Im Falle des zweiten erfindungsgemäßen Herstellungsverfahrens lag die Temperatur bei 150 °C.

Abschließend wird das so erhaltene Produkt abgekühlt und kann entsprechend den Anforderungen an die Weiterverarbeitung konfektioniert werden. Das so erzeugte, erfindungsgemäße Vlies kann ein Flächengewicht im Bereich von 40 bis 150 g/m$^2$ haben, vorzugsweise 50-90g/m$^2$

Vergleich der hergestellten Vliese

Nachfolgend werden die hergestellten erfindungsgemäßen Vliese - Vlies 1 und Vlies 2 - mit dem Vlies aus dem Stand der Technik - Sawasoft® 1236 - verglichen.

Die Prüfergebnisse sind in der unten gezeigten Tabelle 1 aufgeführt. Die dabei ermittelten Parameter wurden gemäß den nachfolgend genannten Prüfmethoden ermittelt:

- Flächengewicht gemäß DIN EN 29073-1

- Dicke gemäß DIN EN ISO 5084

- Dickenquotient: Materialdicke in mm pro 10 g/m$^2$ Vliesstoff

- Zugversuch gemäß DIN EN ISO 9073-03

- Sauggeschwindigkeit gemäß NWSP 010.1.R0 (20) (modifizierte Methode wird unten beschrieben)

- Run Off gemäß NWSP 80.9 R1 (19) (die hierzu zusätzlich modifizierte Methode wird unten beschrieben)

- Stauchhärte 40% nach DIN ISO 3386-1

**Beschreibung der Messmethode für den Run-In**

Die Prüfnorm NWSP 080.9.R1 (19) beschreibt den Nonwoven Run-Off. Als Run-Off wird die Flüssigkeitsmenge definiert, die nicht vom Vliesprüfling aufgenommen wird und am unteren Ende der Probe ausläuft. Diese Menge wird ausgewogen und ins Verhältnis zur aufgebrachten Flüssigkeitsmenge gesetzt. Die Details sind in der Prüfnorm beschrieben.

In Analogie zu dieser Prüfnorm, wird unter Änderung der Neigung des Ablauftisches von 25° auf 45° ein Run-In gemessen, der wie folgt definiert wird: Der Run-In ist die Flüssigkeitsmenge, die 30 s nach dem Flüssigkeitsauftrag auf die Vliesprobe in dieser Vliesprobe verbleibt, also die weder ausläuft noch von dem untergelegten Absorptionsmedium aufgenommen wird. Der Run-In wird gemessen, in dem das Vlies nach der definierten Wartezeit von 30 s vom geneigten Ablauftisch und dem untergelegten Absorptionsmedium entfernt und ausgewogen wird. Die Auswaage entspricht der Gesamtmasse aus Vlies und aufgenommener Flüssigkeit. Nach Abzug der vorher bestimmten Trockenmasse des Vliesprüflings wird die aufgenommene Flüssigkeitsmenge zu dieser Trockenmasse in Bezug gesetzt. Die Berechnung geschieht nach folgender Formel:

$$\text{Run-In} = (m_2 - m_1) \: / \: m_1$$

mit

$m_2 = $ Masse des Vliesprüfling mit der aufgenommenen Menge Flüssigkeit nach 30 s Wartezeit

$m_1 = $ Trockenmasse des Vliesprüflings

Der Run-In kann in g/g oder in % aufgenommener Flüssigkeit angegeben werden. Der Run-In charakterisiert die Zwischenspeicherfähigkeit eines Vliesstoffes bei punktueller Beaufschlagung mit einer bestimmten Flüssigkeitsmenge.

**Beschreibung der Messmethode für den kapillaren Flüssigkeitseinzug**

Die Prüfnorm NWSP 010.1.R10 (20) beschreibt drei Testmethoden für die Nonwoven Absorption, u. a. die als Steighöhenmessung bekannte Methode des kapillaren Flüssigkeitseinzuges, deren Durchführung unter Kapitel 8.3 dieser Norm beschrieben wird.

In Erweiterung dieser Messmethode werden zusätzlich zu den Steighöhen nach definierten Zeiten die Massen der kapillar aufgenommenen Flüssigkeit bestimmt. Dazu wird das Vorratsgefäß mit der Steighöhenflüssigkeit auf eine Waage gestellt, so dass nach Eintauchen des Vliesprüflings die angesaugte Flüssigkeit als negatives Gewicht gemessen werden kann. Dazu wird die Waage, auf dem sich das Gefäß mit der Flüssigkeit befindet, vor dem Eintauchen des Prüflings auf Null tariert. Nach Eintauchen des Prüflings werden, wie in der Norm beschrieben, die Steighöhen nach definierten Zeiten abgelesen und parallel dazu die Gewichtsanzeigen an der Waage abgelesen. Da die Flüssigkeit aus dem Vorratsgefäß vom Vliesstoff herausgesaugt wird, nimmt das Gewicht des Vorratsgefäßes ab, d. h. die Anzeige geht zu negativen Werten.

Durch die Benetzung des Vliesstoffes bildet sich von der Flüssigkeitsoberfläche ausgehend am Rand des Prüflings ein Meniskus aus. Die so entstandene Flüssigkeitslamelle, die infolge der Oberflächenspannung entsteht, zieht zusätzlich als negatives Gewicht an der Waage und muss berücksichtigt werden. Sie kann näherungsweise, aber für Belange der Ergebnisinterpretation präzise genug wie folgt bestimmt werden: Der Prüfling wird so lange in der Flüssigkeit belassen, bis sich die Steighöhe innerhalb von 3 Minuten um keinen Millimeter mehr ändert, also praktisch kapillares Gleichgewicht angenommen werden kann. Sodann wird der Vliesstreifen aus der Flüssigkeit gezogen, die Waage auf null tariert und der Vliesstreifen an die Oberfläche geführt, bis die Flüssigkeit den Vliesstreifen berührt. In dem Moment springt die Flüssigkeitslamelle infolge der Oberflächenspannung an den Vliesstreifen, ohne das Flüssigkeit vom Vlies aufgenommen oder abgegeben wird. Die Anzeige an der Waage gibt in guter Näherung das Gewicht der Flüssigkeitslamelle wieder, um das die während der Messung generierten Werte korrigiert werden.

Die Auswertung geschieht nachfolgender Formel:

$$\text{Kapillarer Einzug} = (m_t - m_L)/m_V$$

mit

$m_t = $ An der Waage abgelesenes Gewicht nach der Zeit t
$m_L = $ Gewicht der Flüssigkeitslamelle, wie oben beschrieben ermittelt

mv = Masse der benetzten Vlieszone, die aus der anhand der Steighöhe ermittelten Fläche und des Flächengewichtes wie folgt Formel ermittelt wird:

$$m_V = h \times b \times FG$$

mit
h = Steighöhe der Flüssigkeit
b = Breite des Vliesprüflings
FG = Flächengewicht des Vliesstoffes in $g/m^2$

Der massenbezogene kapillare Einzug gibt realistischer die zeitabhängige Flüssigkeitsaufnahme wieder als die
Steighöhe.

*Tabelle 1: Eigenschaften der erfindungsgemäßen Vliese im Vergleich zum Stand der Technik*

| | Norm | Sawasoft® 1236 | Vlies 1 | Vlies 2 |
|---|---|---|---|---|
| Flächengewicht | DIN EN 29073-1 | $50 g/m^2$ | $61,6 g/m^2$ | $83,5 \ g/m^2$ |
| Dicke bei 0,5kPa Vorlast | DIN EN ISO 5084 | 0,61 | 1,93mm | 2,40mm |
| Dickenquotient | errechnet | 0,12 mm pro $10 g/m^2$ | 0,31mm pro $10 g/m^2$ | 0,29mm pro $10 g/m^2$ |
| Run Off | NWSP 80.9 R1 (19) | 0% | 0% | 0% |
| Run In | In Anlehnung an NWSP 80.9 R1 (19) | 4,4% | 19,4% | 15,4% |
| Stauchhärte 40% | DIN ISO 3386-1 | 17,3N | 8,9N | 7,3N |
| Quantitative Absorption vertikal nach 20s (kapillarer Flüssigkeitseinzug) | In Anlehnung an NWSP 010.1.R0 (20) | 720% | 3243% | 2071 % |
| Dehnung bei 5N längs auf 1inch Probenbreite | DIN EN ISO 9073-03 | 1,2% | 9,4% | 7,7% |

Die Prüfergebnisse zeigen, dass durch das erfindungsgemäße Verfahren die Zwischenspeicherfähigkeit (Run
In), der Polstereffekt (Stauchhärte) und die Flüssigkeitsaufnahmefähigkeit (Quantitative Absorption) gegenüber dem
Stand der Technik wesentlich verbessert werden können.

**Patentansprüche**

1. Verfahren zur Herstellung eines Aufnahme- und Verteilvliesstoff aus Stapelfasern und Saugmaterial für persönliche
Hygieneprodukte, wobei der Vliesstoff aus thermoplastischen, synthetischen Stapelfaser als Stützfasern, wobei die
Stützfasern homo- oder bikomponente, thermoplastische Polymerfasern sind, die schmelzbare Anteile aufweisen,
Stapelfasern aus thermoplastischen und/oder duroplastischen Polymeren als Verteilfasern und Saugmaterial aus
regenerierter Zellulose zusammengesetzt wird, wobei der Vliesstoff mechanisch verfestigt und anschließend mittels
nachgeschalteter thermischer Aktivierung gebunden wird, **dadurch gekennzeichnet, dass**

- der Vliesstoff aus 5 bis 35 Massenprozent an Stützfasern, 30 bis 90 Massenprozent an Verteilfasern und 5 bis 35
Massenprozent an Saugmaterial zusammengesetzt wird und
- die mechanische Verfestigung mittels Vernadelung erfolgt.

2. Verfahren gemäß Anspruch 1, **dadurch gekennzeichnet, dass** die Vernadelung mit mindestens 200 wirksamen
Kerben/$cm^2$ Vliesstoff, vorzugsweise mit 200 bis 1000 wirksamen Kerben/$cm^2$ Vliesstoff, weiter vorzugsweise mit 300
bis 600 wirksamen Kerben/$cm^2$ Vliesstoff, besonders bevorzugt mit 400 bis 500 wirksamen Kerben/$cm^2$ Vliesstoff,
erfolgt.

3. Verfahren gemäß Anspruch 2, **dadurch gekennzeichnet, dass** einzelne zur Vernadelung verwendete Nadeln zwischen 1 und 9, vorzugsweise zwischen 3 und 6, Kerben und/oder einen Durchmesser zwischen 30 bis 46 Gauge, vorzugsweise zwischen 38 bis 40 Gauge, und eine Länge von 2 bis 3 Zoll aufweisen.

4. Verfahren gemäß Anspruch 2 oder 3, **dadurch gekennzeichnet, dass** die Vernadelung mit mindestens zwei, vorzugsweise vier, Nadelbrettern erfolgt, die beidseitig alternierend in das einlaufende Vlies einstechen, wobei die Nadelbretter vorzugsweise eine Nadeldichte von 12000 bis 80000 Nadeln/m Arbeitsbreite, besonders bevorzugt eine Nadeldichte von 20000 bis 50000 Nadeln/m Arbeitsbreite aufweisen.

5. Verfahren gemäß einem der Ansprüche 1 bis 5, **dadurch gekennzeichnet, dass** die Fasern des Vliesstoffs vor der mechanischen Verfestigung homogen miteinander vermischt werden und mittels eines Kardierverfahrens zu einem Faserflor geformt werden.

6. Verfahren gemäß Anspruch 5, **dadurch gekennzeichnet, dass** der Faserflor in Längsrichtung abgelegt wird.

7. Verfahren gemäß Anspruch 5, **dadurch gekennzeichnet, dass** der Faserflor als ein Lagenaufbau abgelegt wird, der eine erste Schichte und eine zweite Schichte aufweist, wobei die erste Schicht in Längsrichtung gelegt wird und die zweite Schicht in Querrichtung oder mit einer isotropen Ausrichtung gelegt wird und auf oder unter die erste Schicht gelegt wird.

8. Verfahren gemäß Anspruch 7, **dadurch gekennzeichnet, dass** der Lagenaufbau zwei erste Schichten aufweist, wobei die zweite Schicht zwischen die zwei ersten Schichten gelegt wird.

9. Verfahren gemäß Anspruch 8, **dadurch gekennzeichnet, dass** die zwei ersten Schichten jeweils einen Flächenmassenanteil von 25-40 % aufweisen und die zweite Schicht einen Flächenmassenanteil von 20 bis 50% aufweist.

10. Verfahren gemäß einem der Ansprüche 1 bis 9, **dadurch gekennzeichnet, dass** der Vliesstoff nach der mechanischen und thermischen Verfestigung eine Materialdicke pro Flächengewicht von mindestens 0,20 mm/10g/m$^2$, besonders bevorzugst von mindestens 0,25 mm/10g/m$^2$, aufweist.

11. Verfahren gemäß einem der Ansprüche 1 bis 10, **dadurch gekennzeichnet, dass** die thermische Aktivierung mittels Heißluftverfestigung erfolgt, wobei der Vliesstoff vorzugsweise während der Heißluftverfestigung für mindestens 20 Sekunden auf eine Temperatur oberhalb eines Schmelzpunkt schmelzbarer Anteile der Stützfasern erhitzt wird.

12. Aufnahme- und Verteilvliesstoff hergestellt nach einem Verfahren gemäß einem der Ansprüche 1 bis 11.

13. Aufnahme- und Verteilvliesstoff gemäß Anspruch 12, **dadurch gekennzeichnet, dass** der Vliesstoff ein Flächengewicht von 40 bis 150 g/m$^2$, vorzugsweise zwischen 50 und100 g/m$^2$, aufweist.

14. Vorrichtung zur Ausführung eines Verfahrens gemäß einem der Ansprüche 1 bis 11.

Europäisches
Patentamt

European
Patent Office

Office européen
des brevets

# EUROPÄISCHER RECHERCHENBERICHT

Nummer der Anmeldung

EP 25 15 5012

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 692 321 B1 (SANDLER AG [DE]) 13. September 2017 (2017-09-13) * Absätze [0001], [0011], [0014] - [0016], [0019], [0026], [0031], [0032], [0041] - [0044] * * Ansprüche 1-4 * ----- | 1-14 | INV. A61F13/537 D04H1/4258 D04H1/485 D04H1/541 |
| X | EP 2 732 800 A1 (SANDLER AG [DE]) 21. Mai 2014 (2014-05-21) * Absätze [0019], [0020], [0022], [0025] - [0027], [0033] * * Tabellen 1,2 * * Ansprüche 1,4-6,8,9,13,16 * ----- | 1-14 | |

RECHERCHIERTE
SACHGEBIETE (IPC)

A61F
D04H

Der vorliegende Recherchenbericht wurde für alle Patentansprüche erstellt

| Recherchenort | Abschlußdatum der Recherche | Prüfer |
|---|---|---|
| München | 12. Juni 2025 | Beins, Ulrika |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 25 15 5012

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-06-2025

| Im Recherchenbericht angeführtes Patentdokument | Datum der Veröffentlichung | Mitglied(er) der Patentfamilie | Datum der Veröffentlichung |
|---|---|---|---|
| EP 2692321 B1 | 13-09-2017 | DE 102012015219 A1 | 06-02-2014 |
| | | EP 2692321 A1 | 05-02-2014 |
| | | PL 2692321 T3 | 28-02-2018 |
| EP 2732800 A1 | 21-05-2014 | DE 102012022347 A1 | 15-05-2014 |
| | | EP 2732800 A1 | 21-05-2014 |
| | | PL 2732800 T3 | 28-02-2018 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2692321 B1

**In der Beschreibung aufgeführte Nicht-Patentliteratur**

- **ALBRECHT, FUCHS**. Kittelmann: Vliesstoffe. Wiley-VCH Verlag, 2000, 291